# EUROPEAN PATENT APPLICATION

(11) **EP 4 748 831 A1**
(43) Date of publication of application: **27.05.2026**
(21) Application number: 24844684.1
(22) Date of filing: 18.07.2024
(51) Int. Cl.: C07C 227/34, C07C 229/34

(54) **METHOD FOR RESOLVING PHENYLALANINE DERIVATIVE, AND INTERMEDIATE**

(30) Priority: 21.07.2023 CN 202310903753; 05.07.2024 CN 202410898850
(71) Applicant: Neuboron Bio-Scitech Co., Ltd., Xiamen City, Fujian Prov. (CN)
(72) Inventor: ZHOU, Ping, Nanjing, Jiangsu 211112 (CN); BIAN, Ya, Nanjing, Jiangsu 211112 (CN); WANG, Wei, Nanjing, Jiangsu 211112 (CN); CHEN, La-mei, Nanjing, Jiangsu 211112 (CN); CHEN, Lian-bao, Nanjing, Jiangsu 211112 (CN); LIU, Yuan-hao, Nanjing, Jiangsu 211112 (CN)
(74) Representative: Gunzelmann, Rainer
(86) International application number: PCT/CN2024/106107
(87) International publication number: WO 2025/020992

(57) **Abstract**

A method for resolving a phenylalanine derivative of formula (I), and an intermediate. The method comprises: a resolving step comprising using a resolving reagent to react with the phenylalanine derivative represented by formula (I) in a reaction solvent to obtain a resolved intermediate, wherein the resolving reagent is selected from N-acetyl-D-phenylalanine and/or (2R,3R)-(-)-dibenzoyl-L-tartaric acid, and the reaction solvent is selected from 90-99% aqueous ethanol, absolute ethyl alcohol, or acetone. The method uses a chiral reagent to perform salt formation and racemate resolution, and has the advantages of being easy to operate, requiring no special production devices, and being easy to scale up production.

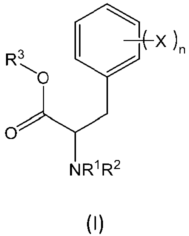

## Description

### Technical Field

The present disclosure relates to the field of medicine, and in particular, to a method for resolving a phenylalanine derivative, and an intermediate.

### Background Art

Boron neutron capture therapy (BNCT) is an effective method for treating tumors. In boron neutron capture therapy, BPA (Chinese name: p-dihydroxyborylphenylalanine or 4-borono-L-phenylalanine) is often used as a drug to treat patients. BPA is transported via the neutral amino acid transporter, namely the L-amino acid transport system (LAT-1). Therefore, only the L-form (S-configuration) of BPA can be used for the treatment of BNCT.

To monitor the distribution of BPA within patients, a radioactive ¹⁸F atom is substituted for one hydrogen atom on the benzene ring of the L-BPA molecule, forming the ¹⁸F-labeled F-BPA. The synthesis of ¹⁸F-BPA requires the use of F-BPA precursors produced in compliance with the requirements of Good Manufacturing Practice of Medical Products (GMP). In addition, phenylalanine derivatives (such as, but not limited to, *tert-butyl* (2S)-2-amino-3-(2,4-dibromophenyl)propionate) are key intermediates required for the production of ¹⁸F-BPA precursors. These intermediate compounds can also serve as synthetic intermediates for boron compounds applicable in BNCT therapy, such as BPA, 2-fluoro-4-borono-L-phenylalanine, and 2,4-diborono-L-phenylalanine, demonstrating very broad application potential.

In the prior art, SFC separation technology is often employed in the production of the aforementioned synthetic intermediates to resolve racemates. This technology suffers from issues such as high equipment costs, small production scale, and poor GMP compliance. Furthermore, due to the *tert-butyl* ester structure of these products, the success rate of the selective asymmetric cleavage of this structure by enzymes is extremely low. Therefore, there is a need to develop a method for the chemical resolution of phenylalanine derivatives that is process-stable, easy to operate, and easy to scale up.

### Summary of the Invention

One object of the present disclosure is to provide a chiral resolution method of a phenylalanine derivative.

Another object of the present disclosure is to provide an intermediate obtained by the chiral resolution method.

In order to achieve the above-mentioned objects, in one aspect, the present disclosure provides a chiral resolution method of a phenylalanine derivative represented by formula (I): 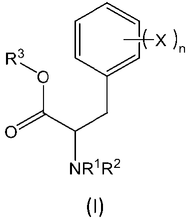
in which X is selected from Br, I, F, Cl, CN, SCN, or OCN;
R¹ and R² are each independently selected from: hydrogen or tert-butoxycarbonyl;
R³ is selected from tert-butyl; and
n = 1 or 2;
wherein the method comprises a resolving step, comprising reacting a resolving agent with the phenylalanine derivative represented by formula (I) in a reaction solvent to obtain a resolution intermediate. The resolving agent is selected from N-acetyl-D-phenylalanine and/or (2R,3R)-(-)-dibenzoyl-L-tartaric acid. The reaction solvent is selected from 90-99% aqueous ethanol, absolute ethanol, or acetone.

Further, the 90-99% aqueous ethanol can be selected from 90% aqueous ethanol, 92% aqueous ethanol, 94% aqueous ethanol, 95% aqueous ethanol, 96% aqueous ethanol, 98% aqueous ethanol, or 99% aqueous ethanol.

In the present disclosure, the phenylalanine derivative represented by formula (I) is a racemate. The method of the present disclosure can effectively perform chiral resolution on the racemate of the phenylalanine derivative to obtain a phenylalanine derivative with a single configuration and high optical purity.

According to some specific embodiments of the present disclosure, the resolving step of the method comprises: reacting the resolving agent with the phenylalanine derivative represented by formula (I) in the reaction solvent, and then crystallizing the reaction system at 10-30°C, followed by filtering to obtain the resolution intermediate. At this point, the product obtained after precipitation and filtration mainly contains a salt of the phenylalanine derivative in the S configuration. Recrystallization can further effectively remove a salt of the phenylalanine derivative in the R configuration from the resolution intermediate.

According to some specific embodiments of the present disclosure, the method comprises stirring the reaction system at 10-30°C for 10-25 h for crystallization, followed by filtering to obtain the resolution intermediate.

According to some specific embodiments of the present disclosure, a compound represented by formula (II) in the resolution intermediate has an ee value of greater than 35%: 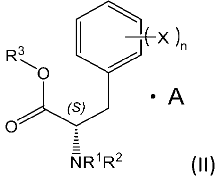 in which A is the resolving agent.

According to some specific embodiments of the present disclosure, the compound represented by formula (II) is selected from a compound represented by formula (II-1) or formula (II-2) as follows:

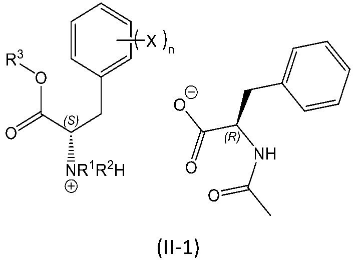

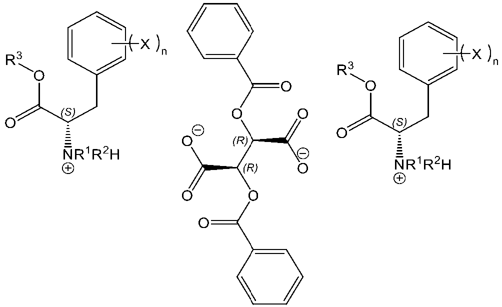

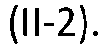

According to some specific embodiments of the present disclosure, the compound represented by formula (II) in the resolution intermediate has an ee value of 36-98%. For example, the compound represented by formula (II) in the resolution intermediate can have an ee value of 36%, 38%, 40%, 45%, 50%, 60%, 70%, 75%, 80%, 85%, 90%, 91%, 92%, 93%, 94%, 95%, 96%, 97%, or 98%.

The compound represented by formula (II) is the salt of the phenylalanine derivative in the S configuration (i.e., the salt obtained by the reaction of the phenylalanine derivative of formula (I) with the resolving agent).

According to some specific embodiments of the present disclosure, reacting the resolving agent with the phenylalanine derivative represented by formula (I) comprises reacting the resolving agent with the phenylalanine derivative represented by formula (I) at 30-55°C to obtain the resolution intermediate.

According to some specific embodiments of the present disclosure, reacting the resolving agent with the phenylalanine derivative represented by formula (I) comprises reacting the resolving agent with the phenylalanine derivative represented by formula (I) at 30-55°C for 2-5 h to obtain the resolution intermediate.

In the present disclosure, the resolution intermediate is a salt formed from the phenylalanine derivative represented by formula (I) with the resolving agent. The phenylalanine derivative contains an amino-containing basic group, which readily reacts with an acidic chiral resolving agent to form a salt of the phenylalanine derivative. The chiral resolving agent can be removed from the resolution intermediate via hydrolysis.

According to some specific embodiments of the present disclosure, the resolution intermediate comprises a salt of the phenylalanine derivative. Further, the resolution intermediate prepared by the method of the present disclosure comprises the salt of the phenylalanine derivative in the S configuration with high optical purity.

According to some specific embodiments of the present disclosure, reacting the resolving agent with the phenylalanine derivative represented by formula (I) comprises reacting the resolving agent with the phenylalanine derivative represented by formula (I) in the reaction solvent; wherein the reaction solvent is 90-99% aqueous ethanol, and the resolving agent is N-acetyl-D-phenylalanine.

According to some specific embodiments of the present disclosure, reacting the resolving agent with the phenylalanine derivative represented by formula (I) comprises reacting the resolving agent with the phenylalanine derivative represented by formula (I) in the reaction solvent; wherein the reaction solvent is acetone, and the resolving agent is (2R,3R)-(-)-dibenzoyl-L-tartaric acid.

According to some specific embodiments of the present disclosure, the molar ratio of the phenylalanine derivative represented by formula (I) to the resolving agent is 1 : 0.4-1.0. Further, the molar ratio of the phenylalanine derivative represented by formula (I) to the resolving agent is 1 : 0.5-0.8.

According to some specific embodiments of the present disclosure, the molar ratio of the phenylalanine derivative represented by formula (I) to the resolving agent is 1 : 0.6.

According to some specific embodiments of the present disclosure, the volume (mL) of the reaction solvent is 10-35 times the mass (g) of the phenylalanine derivative represented by formula (I). For example, the volume (mL) of the reaction solvent can be 10 times, 12 times, 15 times, 18 times, 20 times, 22 times, 25 times, 30 times, 32 times, or 35 times the mass (g) of the phenylalanine derivative represented by formula (I). In other words, the volume-to-mass ratio (mL/g) of the reaction solvent to the phenylalanine derivative represented by formula (I) is 10-35.

According to some specific embodiments of the present disclosure, the mass of the phenylalanine derivative represented by formula (I) used in the resolving step is 30 mg-50 g. For example, the mass of the phenylalanine derivative can be 30 mg, 50 mg, 100 mg, 200 mg, 300 mg, 500 mg, 1 g, 2 g, 5 g, 10 g, 20 g, 40 g, or 50 g. The resolution method of the present disclosure can be suitable for large-scale production.

According to some specific embodiments of the present disclosure, the method further comprises: a step of subjecting the product (resolution intermediate) obtained from the resolving step to at least one recrystallization in a recrystallization solvent to obtain a recrystallized resolution intermediate. In the present disclosure, after the recrystallization treatment, the optical purity of the salt of the phenylalanine derivative in the S configuration can be further increased.

According to some specific embodiments of the present disclosure, the recrystallization comprises stirring the product obtained from the resolving step in the recrystallization solvent at 45-55°C for 1.5-2.5 h, followed by stirring at 15-25°C for 14-20 h, to obtain the recrystallized resolution intermediate.

According to some specific embodiments of the present disclosure, in the recrystallized resolution intermediate, the content of the salt of the phenylalanine derivative in the S configuration (the salt formed from the phenylalanine derivative with the resolving agent) as determined by HPLC is not less than 99%.

According to some specific embodiments of the present disclosure, the recrystallization comprises mixing the resolution intermediate with the recrystallization solvent to obtain a suspension, heating the suspension to 45-55°C within 20-40 min, followed by stirring for 1.5-2.5 h, then cooling to 15-25°C at 4-6°C/h, and stirring for 14-20 h, to obtain the recrystallized resolution intermediate.

According to some specific embodiments of the present disclosure, the recrystallization solvent is selected from at least one of absolute ethanol, tetrahydrofuran, acetonitrile, acetone, and 90-99% aqueous ethanol.

According to some specific embodiments of the present disclosure, the volume (mL) of the recrystallization solvent is 9-15 times the mass (g) of the resolution intermediate. Further, the volume (mL) of the recrystallization solvent can be 9 times, 10 times, 11 times, 12 times, 13 times, 14 times, or 15 times the mass (g) of the resolution intermediate. In other words, the volume-to-mass ratio (mL/g) of the recrystallization solvent to the resolution intermediate is 9-15.

According to some specific embodiments of the present disclosure, the recrystallization further comprises filtering the precipitated crystals after the stirring is completed to obtain a filter cake.

Further, the filter cake obtained after recrystallization is dried under vacuum at 50 ± 5°C to obtain the product. At this point, the optical purity of the salt of the phenylalanine derivative in the S configuration in the product is not less than 99%.

According to some specific embodiments of the present disclosure, the method further comprises a step of hydrolyzing the resolution intermediate obtained from the step of reaction with the resolving agent, or the recrystallized resolution intermediate.

According to some specific embodiments of the present disclosure, a phenylalanine derivative of formula (III) is obtained after hydrolysis: 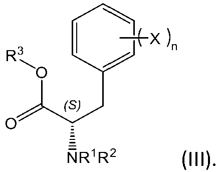 In formula (III), X is independently selected from Br, I, F, Cl, CN, SCN, or OCN; R¹ and R² are each independently selected from: hydrogen or tert-butoxycarbonyl; R³ is selected from tert-butyl; and n = 1 or 2.

In the present disclosure, the phenylalanine derivative of formula (III) is a phenylalanine derivative in the S configuration.

According to some specific embodiments of the present disclosure, the phenylalanine derivative obtained after hydrolysis is selected from the following structures:

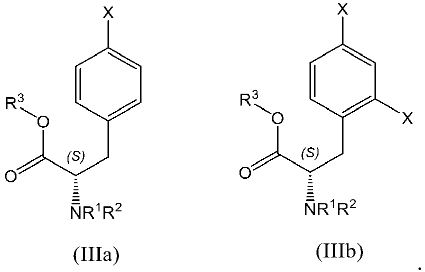

In formula (Illa) and formula (IIIb), X is independently selected from Br, I, F, Cl, CN, SCN, or OCN; R¹ and R² are each independently selected from: hydrogen or tert-butoxycarbonyl; and R³ is selected from tert-butyl.

The hydrolysis can be carried out with reference to conventional hydrolysis in the art. According to some specific embodiments of the present disclosure, the method comprises hydrolyzing the resolution intermediate obtained from the resolving reaction step, or the recrystallized resolution intermediate under an alkaline condition.

According to some specific embodiments of the present disclosure, the pH value of the alkaline condition is 7-14.

According to some specific embodiments of the present disclosure, the alkaline condition is in the presence of a base. Further, the base is selected from, but not limited to, at least one of a metal hydroxide, a metal carbonate, a metal bicarbonate, and aqueous ammonia.

According to some specific embodiments of the present disclosure, the base is selected from one or a mixture of more of sodium hydroxide, potassium hydroxide, sodium carbonate, potassium carbonate, sodium bicarbonate, potassium bicarbonate, or aqueous ammonia.

According to some specific embodiments of the present disclosure, the reaction temperature of the hydrolysis is 0-100°C; preferably 10-80°C; more preferably 15-60°C; and most preferably 20-40°C.

According to some specific embodiments of the present disclosure, the reaction time of the hydrolysis is 5 min-2 h; preferably 10 min-1.5 h; and more preferably 20 min-1 h.

According to some specific embodiments of the present disclosure, the reaction solvent for the hydrolysis is selected from one or a mixture of more of 95% aqueous ethanol, absolute ethanol, water, or acetone.

According to some specific embodiments of the present disclosure, the phenylalanine derivative is selected from the following structures: 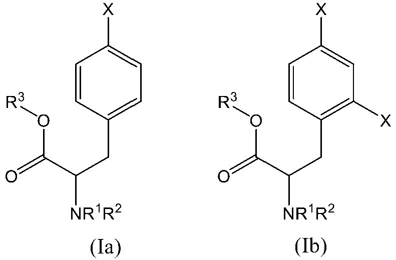
in which X is independently selected from Br, I, F, Cl, CN, SCN, or OCN;
R¹ and R² are each independently selected from: hydrogen or tert-butoxycarbonyl; and
R³ is selected from tert-butyl.

According to some specific embodiments of the present disclosure, X is independently selected from Br, I, F, or Cl.

According to some specific embodiments of the present disclosure, the phenylalanine derivative is selected from, but not limited to, one of the following structures:

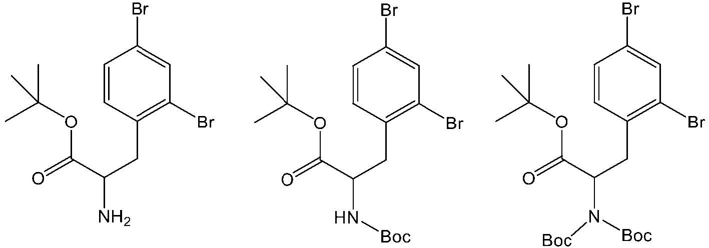

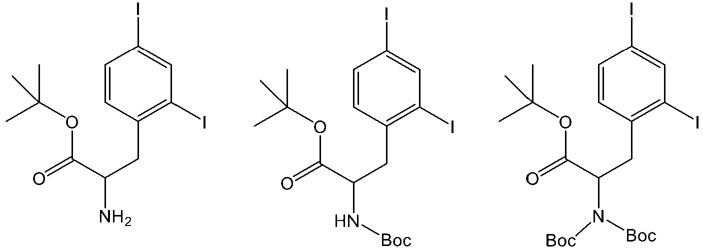

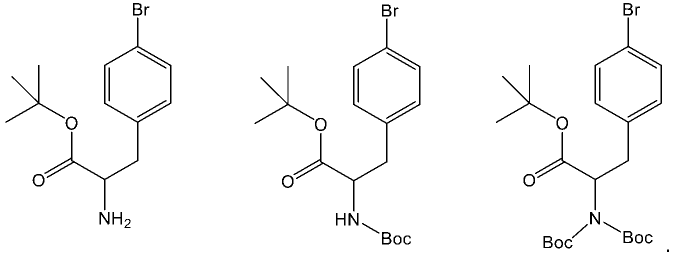

*Tert-butyl* 2-amino-3-(2,4-dibromophenyl)propionate is provided as an example in the present disclosure, and its resolution method is described in detail as follows.

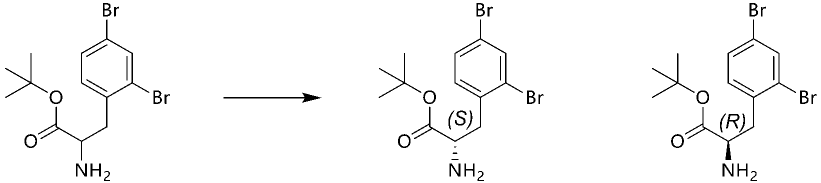

An appropriate amount of *tert-butyl* 2-amino-3-(2,4-dibromophenyl)propionate is added to a reaction vessel, followed by 10-30 v of a reaction solvent for dissolution. Then, a resolving agent is added to give a suspension. The suspension is stirred at 30-55°C for 2-5 h, cooled to 20°C, and maintained at this temperature for 10-20 h to precipitate crystals. The crystals are subjected to suction filtration to give a crude product. The crude product is placed in a reaction vessel, and 12 ± 2 v of acetone is added at room temperature to give a suspension. The suspension is heated to 50 ± 5°C within half an hour, and stirred for 2 ± 0.5 h while maintaining at this temperature. The suspension is cooled to 20 ± 5°C at 5 ± 1°C/h, and stirred for about 17 ± 3 h while maintaining at this temperature to precipitate crystals, i.e., the recrystallized product, from which some impurities are removed. The crystals are subjected to suction filtration at room temperature. The filter cake is dried under vacuum at 50 ± 5°C to give a chiral acid salt of *tert-butyl* (2S)-2-amino-3-(2,4-dibromophenyl)propionate. The resulting chiral acid salt can be neutralized with a base until the pH value of the reaction solution reaches 7-14, followed by freeing and filtering to give *tert-butyl* (2S)-2-amino-3-(2,4-dibromophenyl)propionate. *Tert-butyl* (2S)-2-amino-3-(2,4-dibromophenyl)propionate:

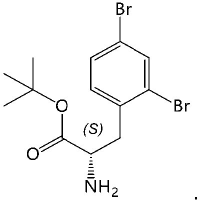

The *tert-butyl* 2-amino-3-(2,4-dibromophenyl)propionate is prepared by the following method.

### 1. Synthesis of tert-butyl 2-amino-3-(2,4-dibromophenyl)propionate

N-Diphenylmethylene-glycine *tert-butyl* ester (40 g, 135.42 mmol, 1 eq), 1,4-dibromobenzyl bromide (44.53 g, 135.42 mmol, 1 eq), and tetrabutylammonium bromide (436.55 mg, 1.35 mmol, 0.01 eq) are dissolved in 300 mL of toluene, and 80 mL aqueous solution of potassium hydroxide (100 g, 1.78 mol, 13.16 eq) is added. The mixture is stirred at 25°C for 12 h until the reaction is completed. The resulting mixture is allowed to stand to separate into layers. The aqueous phase is discarded, and the organic phase is retained. The organic phase is concentrated at 55-60°C until no distillate emerges. Acetonitrile (6 v) is added, and the mixture is heated to 50-55°C to give a clear solution. The solution is cooled at a slow gradient (10-15°C per hour) to 0-5°C for crystallization. The solution is stirred at 0-5°C for 1-2 h, and filtered, and the filter cake is rinsed with acetonitrile (0-5°C, 1 v). The mother liquor is discarded, and the filter cake is dried in a blast drying oven at 45-50°C to give N-diphenylmethylene-2,4-dibromophenylalanine *tert-butyl* ester.

N-Diphenylmethylene-2,4-dibromophenylalanine *tert-butyl* ester and tetrahydrofuran (3 v) are added to a 50.00 ml four-necked flask. A mixed solution of citric acid (4.00 eq.) and water (3 v) is added dropwise. The mixture is stirred for 20 h, and the reaction solution is concentrated at 45-55°C until no distillate emerges. Ethyl acrylate (5 v) and water (5 v) are added and stirred for 0.5 h. The layers are separated, and the aqueous phase is retained. The organic phase is extracted with water (3.5 v) twice. The aqueous phases are combined and extracted with EA (1.5 v) twice. EA (5 v) is added to the aqueous phase with stirring, and the pH is adjusted to 8-9 with sodium carbonate. The organic phase is washed with a saturated sodium chloride solution (5 v) once, and concentrated at 45-55°C until no distillate emerges, to give *tert-butyl* 2-amino-3-(2,4-dibromophenyl)propionate as an oil (51.87 g, purity: 98.0%, yield: 70.5%).

In another aspect, the present disclosure also provides an intermediate represented by formula (II): 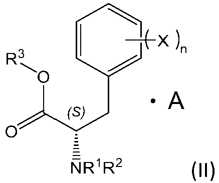
in which X is selected from Br, I, F, Cl, CN, SCN, or OCN;
R¹ and R² are each independently selected from: hydrogen or tert-butoxycarbonyl;
R³ is selected from tert-butyl;
n = 1 or 2; and
A is a resolving agent.

Further, A is independently selected from N-acetyl-D-phenylalanine or (2R,3R)-(-)-dibenzoyl-L-tartaric acid.

According to some specific embodiments of the present disclosure, the compound represented by formula (II) is selected from a compound represented by formula (II-1) or formula (II-2) as follows:

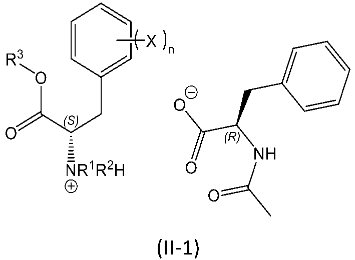

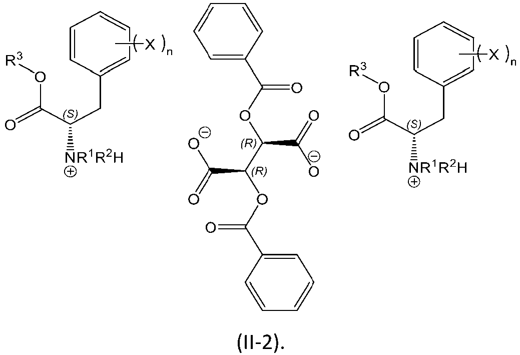

According to some specific embodiments of the present disclosure, the intermediate comprises one of the following structures:

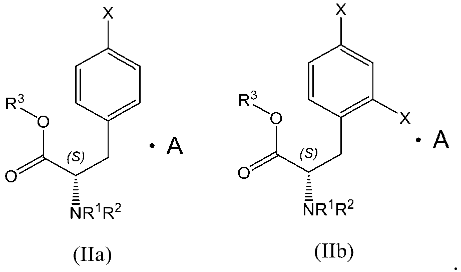

According to some specific embodiments of the present disclosure, X is independently selected from Br, I, F, or Cl.

The intermediate represented by formula (II) can be prepared by the chiral resolution method of the present disclosure.

The phenylalanine derivative of formula (III) can be prepared by the chiral resolution method of the present disclosure.

The intermediate represented by formula (II) can be used to prepare the phenylalanine derivative of formula (III) of the present disclosure.

The intermediate represented by formula (II) and/or the phenylalanine derivative represented by formula (III) of the present disclosure can serve as synthetic intermediates for preparing boron compounds applicable in BNCT therapy, such as 4-borono-L-phenylalanine (BPA), 2-fluoro-4-borono-L-phenylalanine (F-BPA), and 2,4-diborono-L-phenylalanine, demonstrating very broad application potential.

In summary, the present disclosure provides a method for resolving a phenylalanine derivative, and an intermediate thereof. The technical solution of the present disclosure has the following advantages:
the present disclosure uses a chiral reagent to resolve racemates via salt formation, and has the advantages of being easy to operate, requiring no special production equipment, and being easy to scale up production.

### Brief Description of the Drawings

FIG. 1 shows the chromatogram of structural identification example 1.
FIG. 2 shows the chromatogram of structural identification example 2.

### Detailed Description of Embodiments

The implementation process and beneficial effects of the present disclosure are described in detail below by way of specific examples, which are intended to help readers better understand the essence and characteristics of the present disclosure and shall not be construed as limiting the scope of implementation of the present disclosure.

Chromatographic conditions for determining ee values in the examples of the present disclosure: chromatographic column: DAICEL CHIRALPAK ZWIX (250 mm * 4 mm, 3 um); mobile phase [water (50 mM formic acid + 25 mM diethylamine): methanol : tetrahydrofuran = 2 : 38 : 60]; flow rate: 0.5 ml/min; detection wavelength: 210 nm; and column temperature: 40°C.

### Example 1:

30 mg of *tert-butyl* 2-amino-3-(2,4-dibromophenyl)propionate was added to a reaction vessel, followed by absolute ethanol (16 v, the volume of absolute ethanol was 16 times the mass of *tert-butyl* 2-amino-3-(2,4-dibromophenyl)propionate), and the mixture was stirred to give a clear solution. N-Acetyl-D-phenylalanine (0.6 eq) was added, and the mixture was stirred at 40°C for 5 h. After the reaction was completed, it was cooled to 10°C to precipitate crystals. The crystals were subjected to suction filtration and then dried under vacuum. The sample was determined to have an ee value of 36.8%.

### Example 2:

This example differed from Example 1 in that acetone was used as the solvent in this example instead of absolute ethanol used in Example 1. The resulting sample had an ee value of 48.2%.

### Example 3:

This example differed from Example 1 in that 95% aqueous ethanol (20 v) was used as the solvent in this example instead of absolute ethanol (16 v) used in Example 1. The resulting sample had an ee value of 86.2%.

### Example 4:

Recrystallization: The sample from Example 3 was placed in a reaction vessel. 95% aqueous ethanol (10 v) was added at room temperature to give a suspension. The suspension was heated to 50°C within half an hour, and stirred for 2 h while maintaining at 50°C. The suspension was cooled to 20°C at 5°C/h, and stirred overnight while maintaining at this temperature. The suspension was subjected to suction filtration at room temperature, and the filter cake was dried under vacuum at 50°C. The sample was determined to have an ee value of 94.6%.

The recrystallized product was hydrolyzed at pH 10-11, followed by freeing and filtering to give *tert-butyl* (2S)-2-amino-3-(2,4-dibromophenyl)propionate.

### Example 5:

This example differed from Example 4 in that acetone (10 v) was used as the recrystallization solvent in this example instead of 95% aqueous ethanol (10 v) used in Example 4. The resulting sample had an ee value of 93.6%.

The recrystallized product was hydrolyzed at pH 7-8, followed by freeing and filtering to give *tert-butyl* (2S)-2-amino-3-(2,4-dibromophenyl)propionate.

### Example 6:

This example differed from Example 4 in that acetonitrile (10 v) was used as the recrystallization solvent in this example instead of 95% aqueous ethanol (10 v) used in Example 4. The resulting sample had an ee value of 87.2%.

The recrystallized product was hydrolyzed at pH 10-11, followed by freeing and filtering to give *tert-butyl* (2S)-2-amino-3-(2,4-dibromophenyl)propionate.

### Example 7:

This example differed from Example 4 in that tetrahydrofuran (10 v) was used as the recrystallization solvent in this example instead of 95% aqueous ethanol (10 v) used in Example 4. The resulting sample had an ee value of 93.6%.

The recrystallized product was hydrolyzed at pH 12-13, followed by freeing and filtering to give *tert-butyl* (2S)-2-amino-3-(2,4-dibromophenyl)propionate.

### Example 8:

This example differed from Example 4 in that absolute ethanol was used as the recrystallization solvent in this example instead of 95% aqueous ethanol (10 v) used in Example 4. The resulting sample had an ee value of 96.2%.

The recrystallized product was hydrolyzed at pH 9-10, followed by freeing and filtering to give *tert-butyl* (2S)-2-amino-3-(2,4-dibromophenyl)propionate.

### Example 9:

30 mg of *tert-butyl* 2-amino-3-(2,4-dibromophenyl)propionate was added to a reaction vessel, followed by acetone (27 v), and the mixture was stirred to give a clear solution. (2R,3R)-(-)-Dibenzoyl-L-tartaric acid (0.6 eq) was added, and the mixture was stirred at 40°C for 5 h. After the reaction was completed, it was cooled to 10°C to precipitate crystals. The crystals were subjected to suction filtration and then dried under vacuum to give a sample.

Recrystallization: The above sample was weighed and placed in a reaction vessel. Tetrahydrofuran (10 v) was added at room temperature to give a suspension. The suspension was heated to 50°C within half an hour, and stirred for 2 h while maintaining at 50°C. The suspension was cooled to 20°C at 5°C/h, and stirred overnight while maintaining at this temperature. The suspension was subjected to suction filtration at room temperature, and the filter cake was dried under vacuum at 50°C. The resulting sample had an ee value of 78.2%.

The recrystallized product was hydrolyzed at pH 10-11, followed by freeing and filtering to give *tert-butyl* (2S)-2-amino-3-(2,4-dibromophenyl)propionate.

### Example 10:

About 600 mg of *tert-butyl* 2-amino-3-(2,4-dibromophenyl)propionate was weighed and placed in a 20 ml glass vial. 19.2 ml of acetone was added at room temperature, and the mixture was stirred to give a clear solution. 0.6 eq of (2R,3R)-(-)-dibenzoyl-L-tartaric acid was weighed and added to the glass vial to give a suspension. The suspension was heated to 50°C within half an hour, and stirred for 2 h while maintaining at 50°C. The suspension was cooled to 20°C at 5°C/h, and stirred for about 23 h while maintaining at this temperature. The suspension was subjected to suction filtration at room temperature, and the filter cake was dried under vacuum at 50°C (ee value: 80.36%, yield: 39.15%).

Recrystallization: The above sample was weighed and placed in a reaction vial, and 12 v of acetone was added at room temperature to give a suspension. The suspension was heated to 50°C within half an hour, and stirred for 2 h while maintaining at 50°C. The suspension was cooled to 20°C at 5°C/h, and stirred for about 17 h while maintaining at this temperature. The suspension was centrifuged, and the filter cake was dried under vacuum (ee value: 96.94%, yield: 92.03%).

The recrystallized product was hydrolyzed at pH 10-11, followed by freeing and filtering to give *tert-butyl* (2S)-2-amino-3-(2,4-dibromophenyl)propionate.

### Example 11:

About 10 g of *tert-butyl* 2-amino-3-(2,4-dibromophenyl)propionate was weighed and placed in a 1 L jacketed reactor. 320 ml of acetone was added at room temperature, and the mixture was stirred to give a clear solution. 0.6 eq of (2R,3R)-(-)-dibenzoyl-L-tartaric acid was weighed, dissolved in a solvent until clear, and then added to the jacketed reactor to give a suspension. The suspension was heated to 50°C within half an hour, and stirred for 2 h while maintaining at 50°C. The suspension was cooled to 20°C at 5°C/h, and stirred for about 17 h while maintaining at this temperature. The suspension was subjected to suction filtration at room temperature, and the filter cake was dried under vacuum at 50°C for about 6 h (ee value: 93.36%, yield: 39.57%).

Recrystallization: About 3 g of the product was weighed and placed in a 50 mL jacketed reactor. 36 ml of acetone was added at room temperature to give a suspension. The suspension was heated to 50°C within half an hour, and stirred for 2 h while maintaining at 50°C. The system was cooled to 20°C at 5°C/h, and stirred for about 17 h while maintaining at this temperature. The system was subjected to suction filtration at room temperature, and the filter cake was dried under vacuum at 50°C (ee value: 97.9%).

The recrystallized product was hydrolyzed at pH 10-11, followed by freeing and filtering to give *tert-butyl* (2S)-2-amino-3-(2,4-dibromophenyl)propionate.

### Example 12:

About 50 g of *tert-butyl* 2-amino-3-(2,4-dibromophenyl)propionate was weighed and placed in a 5 L jacketed reactor. 1600 ml of acetone was added at room temperature, and the mixture was stirred to give a clear solution. 0.6 eq of (2R,3R)-(-)-dibenzoyl-L-tartaric acid was weighed, dissolved in a solvent until clear, and then added to the jacketed reactor to give a suspension. The suspension was heated to 50°C within half an hour, and stirred for 2 h while maintaining at 50°C. The suspension was cooled to 20°C at 5°C/h, and stirred for about 20 h while maintaining at this temperature. The suspension was subjected to suction filtration at room temperature, and the filter cake was dried under vacuum at 50°C for about 10 h (ee value: 90.52%, yield: 40.38%).

Recrystallization: About 15 g of the product was weighed and placed in a 250 mL jacketed reactor. 225 ml of acetone was added at room temperature to give a suspension. The suspension was heated to 50°C within half an hour, and stirred for 2 h while maintaining at 50°C. The suspension was cooled to 20°C at 5°C/h, and stirred for about 17 h while maintaining at this temperature. The suspension was subjected to suction filtration at room temperature, and the filter cake was dried under vacuum at 50°C (ee value: 99.3%).

The recrystallized product was hydrolyzed at pH 10-11, followed by freeing and filtering to give *tert-butyl* (2S)-2-amino-3-(2,4-dibromophenyl)propionate.

### Comparative example 1:

1.0 g of *tert-butyl* 2-amino-3-(2,4-dibromophenyl)propionate was added to a reaction vessel, followed by 8 ml of methyl *tert-butyl* ether. The mixture was stirred at 30-35°C for 12 h until the sample was dissolved to be clear. 1.0 eq of (R)-(-)-mandelic acid (1.0 eq) was added, and the mixture was stirred at 40-45°C for another 2 h and then cooled to 10°C. The sample was in the form of a suspension, and was centrifuged and sampled for testing (ee: 0%).

### Comparative example 2:

1.0 g of *tert-butyl* 2-amino-3-(2,4-dibromophenyl)propionate was added to a reaction vessel, followed by 12 ml of a methyl *tert-butyl* ether-methanol (5 : 1) solution. After the mixture was stirred to give a clear solution, 0.5 eq of D-tartaric acid was added, and the mixture was stirred at room temperature for 12 h. The sample was dissolved to be clear. Upon being cooled to 10°C, the sample was dissolved to be clear, and no crystals precipitated.

### Comparative example 3:

Comparative example 3 differed from Example 1 in that acetonitrile was used as the solvent in Comparative example 3 instead of absolute ethanol used in Example 1. The resulting sample had an ee value of 6.0%.

### Comparative example 4:

Comparative example 4 differed from Example 1 in that L-camphorsulfonic acid (0.6 eq) was used as the resolving agent and acetone (5 v) as the solvent respectively in Comparative example 4, instead of N-acetyl-D-phenylalanine (0.6 eq) and absolute ethanol (16 v) used respectively in Example 1. The resulting sample had an ee value of 6.2%.

### Comparative example 5:

Comparative example 5 differed from Comparative example 4 in that acetonitrile (29 v) was used as the solvent in Comparative example 5 instead of acetone (5 v) used in Comparative example 4. The resulting sample had an ee value of 0%.

### Comparative example 6:

Comparative example 6 differed from Comparative example 4 in that L-cysteine (0.6 eq) was used as the resolving agent in Comparative example 6 instead of L-camphorsulfonic acid (0.6 eq) used in Comparative example 4. The resulting sample had an ee value of 16.8%.

### Comparative example 7:

Comparative example 7 differed from Comparative example 6 in that 95% aqueous ethanol (5 v) was used as the solvent in Comparative example 7 instead of acetone (5 v) used in Comparative example 6. The resulting sample had an ee value of 0%.

### Structural identification example 1:

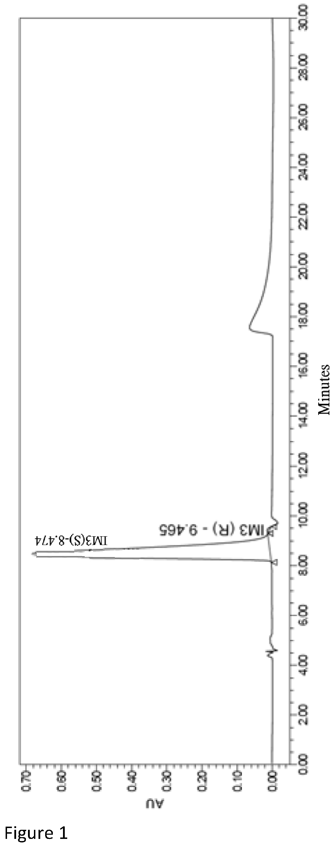

The sample prepared in Example 12 was taken and tested under the aforementioned conditions for determining ee values. The results are shown in FIG. 1 and Table 1. FIG. 1 shows the chromatogram, and Table 1 shows the peak table corresponding to the chromatogram shown in FIG. 1.

**Table 1**

| Item | Peak Name | RT | Area | %Area | Height |
|---|---|---|---|---|---|
| 1 | IM3 (S) | 8.474 | 18567962 | 99.65 | 678302 |
| 2 | IM3 (R) | 9.465 | 66079 | 0.35 | 6541 |

Referring to FIG. 1, the peak at 8.474 min is the chromatographic peak of the S-configuration product (i.e., IM3 (S)), accounting for 99.65% (see Table 1); and the peak at 9.465 min is the chromatographic peak of the R-configuration product (i.e., IM3 (R)), accounting for only 0.35% (see Table 1). It can be seen that the method of the present disclosure can effectively resolve the S-configuration product.

### Structural identification example 2:

The product prepared in Example 8 of the present disclosure and ethyl acetate (5 v) were added to a reaction vessel, to which (Boc)₂O (6.00 eq.) and DMAP (0.01 eq.) were added at 20-25°C. The mixture was stirred at 55-60°C for 6 h. 2N aqueous ammonia (5 v) was added, and the mixture was stirred for 12 h. The layers were separated. A saturated ammonium chloride solution (5 v) was added to the organic phase, and the mixture was stirred for 0.5 h. The layers were separated. The organic phase was concentrated at 40-45°C until no distillate emerged, and n-heptane (1 v) was added for azeotropic distillation once. n-Heptane (5 v) was added, and the mixture was cooled to 0-5°C and filtered, and the mother liquor was retained. A 5% citric acid solution (5 v) was added to the mother liquor, and the mixture was cooled to 0-5°C, stirred for 0.5 h, and allowed to stand to separate into layers. The organic phase was concentrated at 85-90°C until no distillate emerged, to give a pale yellow solid. Ethyl acetate (10 v) was added, and the mixture was purged with nitrogen once and stirred. AcOK (3.50 eq.) and B₂Pin₂ (2.50 eq.) were added, and the mixture was purged with nitrogen once and stirred. Pd(dppf)Cl₂ (0.10 eq.) was added, and the mixture was purged with nitrogen three times. The mixture was stirred at 75-78°C for 17 h. Under nitrogen atmosphere, the mixture was cooled to 20-25°C and filtered through celite pad, and the filter cake was washed with EA (1 v) once and discarded. Under nitrogen atmosphere, the mother liquor was filtered through silica gel pad, and the filter cake was washed with EA (1 v) once and discarded. Activated carbon (1.0 X) and silica gel (1.0 X) were added to the filtrate, and the mixture was heated to 55-60°C and stirred for 0.5 h. The mixture was cooled to 20-25°C and filtered, and the filter cake was discarded. The mother liquor was washed with a saturated ammonium chloride solution once, and the aqueous phase was discarded. The mother liquor was concentrated at 50-55°C until no distillate emerged. n-Heptane (2 v) was added for azeotropic distillation once. Silica gel (3.0 X) and n-heptane (5 v) were added, and the mixture was concentrated until no distillate emerged. The mixture was subjected to suction filtration through silica gel pad. The mother liquor was retained, and the filter cake was discarded. The mother liquor was concentrated at 50-55°C until no distillate emerged. n-Heptane (5 v) was added to give a clear solution. The solution was cooled to -10-0°C for crystallization. The solution was dried in an oven at 30-35°C for 12 h to give N,N-di(tert-butoxycarbonyl)-2,4-di(4,4,5,5-tetramethyl-1,3,2-dioxaborolan-(25)-2-yl)-phenylalanine *tert-butyl* ester as a white solid.

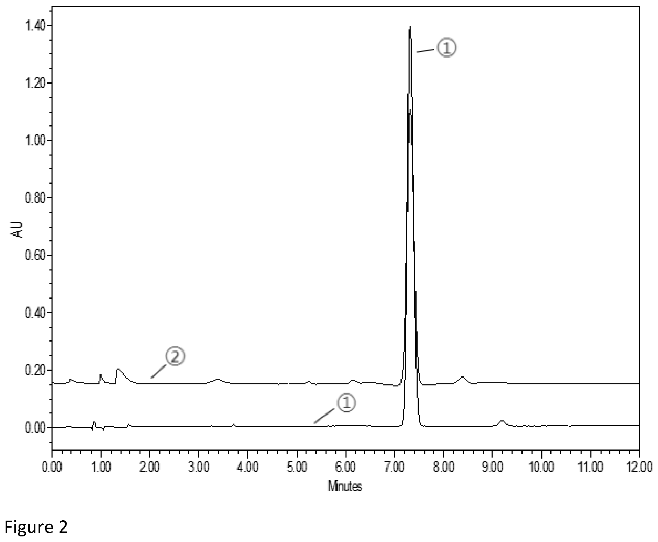

The resulting white solid (N,N-di(tert-butoxycarbonyl)-2,4-di(4,4,5,5-tetramethyl-1,3,2-dioxaborolan-(2S)-2-yl)-phenylalanine *tert-butyl* ester) and a reference standard of N,N-di(tert-butoxycarbonyl)-2,4-di(4,4,5,5-tetramethyl-1,3,2-dioxaborolan-(25)-2-yl)-phenylalanine *tert-butyl* ester [batch number: EW22935-28-P1(P2)-peak2-L; source: WuXi AppTec (Wuhan) Co., Ltd.] were injected into the supercritical fluid chromatographic column according to the chromatographic conditions as shown in Table 2, respectively. The chromatogram is shown in FIG. 2.

**Table 2**

| | | | |
|---|---|---|---|
| Chromatographic Column | **(R,R) Whelk-O1 (4.6 * 150 mm, 3.5 µm)** | | |
| Detection Wavelength (DAD) | 210 nm | | |
| Column Temperature (°C) | 37°C | | |
| Flow Rate (ml/min) | 2.0 ml/min | | |
| Acquisition Time (min) | 12 min | | |
| Injection Volume | 5 µl | | |
| Injection Concentration | 0.5 mg/ml | | |
| Mobile Phase A | CO₂ | | |
| Mobile Phase B | 0.1% Aqueous ammonia-isopropanol solution (v/v) | | |
| Diluent | Methanol | | |
| Isocratic Elution | Time (min) | Mobile phase A, % | Mobile phase B, % |
| | 0.00 | 95.0 | 5.0 |
| | 12.00 | 95.0 | 5.0 |

Referring to FIG. 2, N,N-di(tert-butoxycarbonyl)-2,4-di(4,4,5,5-tetramethyl-1,3,2-dioxaborolan-(2S)-2-yl)-phenylalanine *tert-butyl* ester prepared from *tert-butyl* (2S)-2-amino-3-(2,4-dibromophenyl)propionate obtained by the resolution method of the present disclosure (see curve ② in FIG. 2) has the same retention time as that of the reference standard (see curve ① in FIG. 2). Since the reference standard is a commercially available S-configuration compound, it is evident that the phenylalanine derivative obtained by the resolution method of the present disclosure is in the S configuration. Obviously, the resolution intermediate and the phenylalanine derivative prepared by the method of the present disclosure are both in the S configuration, and thus the present disclosure can effectively isolate the S-configuration products.

In summary, the resolution method of the present disclosure can not only effectively resolve the racemates to isolate and obtain the desired S-configuration compounds, but also be suitable for large-scale production with a relatively low cost, demonstrating significant beneficial effects.

## Claims

1. A chiral resolution method of a phenylalanine derivative represented by formula (I): 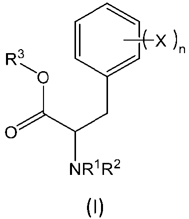
in which X is selected from Br, I, F, Cl, CN, SCN, or OCN;
R¹ and R² are each independently selected from: hydrogen or tert-butoxycarbonyl;
R³ is selected from tert-butyl; and
n = 1 or 2;
wherein the method comprises a resolving step, comprising reacting a resolving agent with the phenylalanine derivative represented by formula (I) in a reaction solvent to obtain a resolution intermediate; wherein the resolving agent is selected from N-acetyl-D-phenylalanine and/or (2R,3R)-(-)-dibenzoyl-L-tartaric acid; and the reaction solvent is selected from 90-99% aqueous ethanol, absolute ethanol, or acetone.

2. The chiral resolution method according to claim 1, wherein the resolving step comprises:
reacting the resolving agent with the phenylalanine derivative represented by formula (I) in the reaction solvent, and then crystallizing the reaction system at 10-30°C, followed by filtering to obtain the resolution intermediate.

3. The chiral resolution method according to claim 2, wherein the method comprises stirring the reaction system at 10-30°C for 10-25 h for crystallization, followed by filtering to obtain the resolution intermediate.

4. The chiral resolution method according to claim 1, wherein a compound represented by formula (II) in the resolution intermediate has an ee value of greater than 35%: 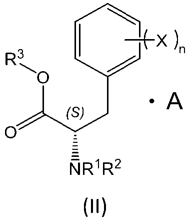 in which A is the resolving agent.

5. The chiral resolution method according to claim 4, wherein the compound represented by formula (II) is selected from a compound represented by formula (II-1) or formula (II-2) as follows:

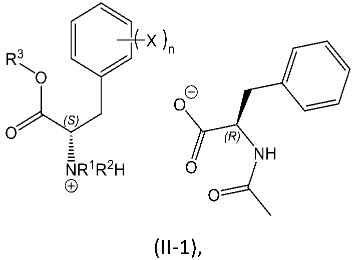

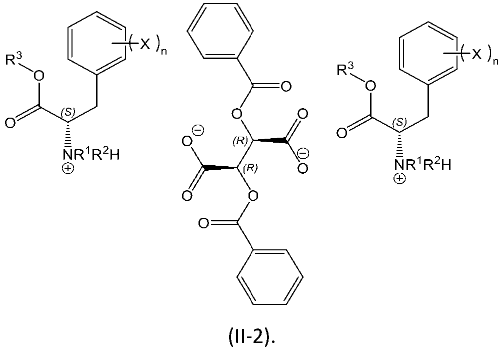

6. The chiral resolution method according to claim 1, wherein reacting the resolving agent with the phenylalanine derivative represented by formula (I) comprises reacting the resolving agent with the phenylalanine derivative represented by formula (I) at 30-55°C to obtain the resolution intermediate.

7. The chiral resolution method according to claim 1, wherein the reaction solvent is 90-99% aqueous ethanol, and the resolving agent is N-acetyl-D-phenylalanine; or
the reaction solvent is acetone, and the resolving agent is (2R,3R)-(-)-dibenzoyl-L-tartaric acid.

8. The chiral resolution method according to claim 1, wherein the molar ratio of the phenylalanine derivative represented by formula (I) to the resolving agent is 1 : 0.4-1.0.

9. The chiral resolution method according to claim 1, wherein the volume (mL) of the reaction solvent is 10-35 times the mass (g) of the phenylalanine derivative.

10. The chiral resolution method according to claim 1, wherein the method further comprises: a step of subjecting the obtained resolution intermediate to at least one recrystallization in a recrystallization solvent.

11. The chiral resolution method according to claim 10, wherein the recrystallization comprises stirring the resolution intermediate obtained from the resolving step in the recrystallization solvent at 45-55°C for 1.5-2.5 h, followed by stirring at 15-25°C for 14-20 h, to obtain a recrystallized resolution intermediate.

12. The chiral resolution method according to claim 10, wherein the recrystallization comprises mixing the resolution intermediate with the recrystallization solvent to obtain a suspension, heating the suspension to 45-55°C within 20-40 min, followed by stirring for 1.5-2.5 h, then cooling to 15-25°C at 4-6°C/h, and stirring for 14-20 h, to obtain the recrystallized resolution intermediate.

13. The chiral resolution method according to claim 10, wherein the recrystallization solvent is selected from at least one of absolute ethanol, tetrahydrofuran, acetonitrile, acetone, and 90-99% aqueous ethanol; and/or
the volume (mL) of the recrystallization solvent is 9-15 times the mass (g) of the resolution intermediate.

14. The chiral resolution method according to claim 1, 10, or 11, wherein the method further comprises a step of hydrolyzing the resolution intermediate obtained from the resolving step or the recrystallized resolution intermediate to obtain a phenylalanine derivative of formula (III) after hydrolysis:

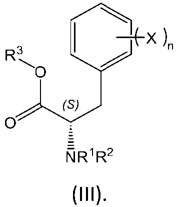

15. The chiral resolution method according to claim 1, wherein the mass of the phenylalanine derivative represented by formula (I) used in the resolving step is 30 mg-50 g.

16. The chiral resolution method according to claim 1, wherein the phenylalanine derivative is selected from the following structures: 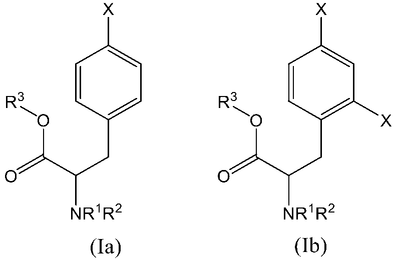
in which X is independently selected from Br, I, F, Cl, CN, SCN, or OCN;
R¹ and R² are each independently selected from: hydrogen or tert-butoxycarbonyl; and
R³ is selected from tert-butyl.

17. The chiral resolution method according to claim 1, wherein the phenylalanine derivative is selected from one of the following structures:

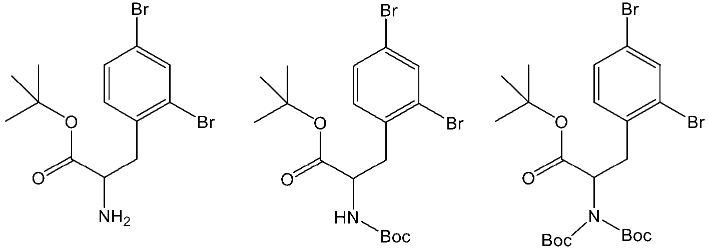

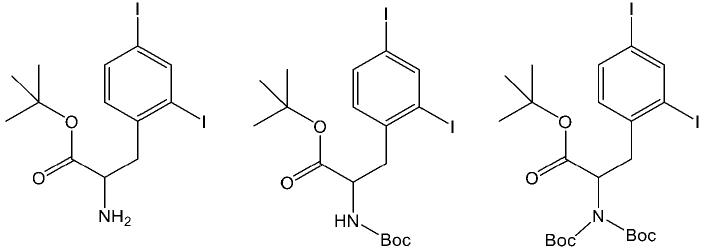

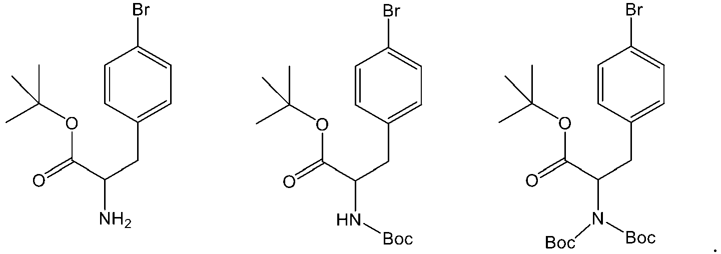

18. An intermediate represented by formula (II): 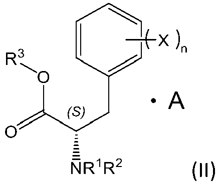
in which X is selected from Br, I, F, Cl, CN, SCN, or OCN;
R¹ and R² are each independently selected from: hydrogen or tert-butoxycarbonyl;
R³ is selected from tert-butyl;
n = 1 or 2; and
A is a resolving agent.

19. The intermediate according to claim 18, wherein A is independently selected from N-acetyl-D-phenylalanine or (2R,3R)-(-)-dibenzoyl-L-tartaric acid.
